# EUROPEAN PATENT SPECIFICATION

(11) **EP 0 292 100 B1**
(45) Date of publication and mention of the grant of the patent: **11.01.1995**
(21) Application number: 88302521.5
(22) Date of filing: 23.03.1988
(51) Int. Cl.: A61K 31/155, A61K 9/12

(54) **Composition for the prophylaxis or treatment of pneumocystis carinii pneumonia**
Zusammensetzung für die Prophylaxe von Pneumocystis-carinii-Pneumonie
Composition pour la prophylaxie de la pneumonie à Pneumocystis carinii

(30) Priority: 26.03.1987 US 30873; 01.03.1988 US 162562
(43) Date of publication of application: 23.11.1988
(73) Proprietor: Sloan-Kettering Institute For Cancer Research, New York, New York 10021 (US)
(72) Inventor: Bernard, Edward M., New York, NY 10021 (US); Armstrong, Donald, New York, NY 10027 (US)
(74) Representative: Wright, Robert Gordon McRae

(56) References cited:
- DE-A- 3 533 494
- CHEMICAL ABSTRACTS, vol. 106, no. 26, 29 June 1987, page 364, abstract no. 219443n, Columbus, Ohio, US; R.J. DEBS et al.: "Selective enhancement of pentamidine uptake in the lung by aerosolization and delivery in liposomes"
- CHEMICAL ABSTRACTS, vol. 106, no. 15, 13 April 1987, page 21, abstract no. 113175m, Columbus, Ohio, US; R.J. DEBS et al.: "Successful treatment with aerosolized pentamidine of pneumocystis carinii pneumonia rats"

## Description

This invention relates to the prophylaxis and treatment of pneumocystis carinii pneumonia (hereinafter referred to as PCP) by the aerosol administration of pentamidine, and compositions for this purpose.

PCP is the most common life-threatening opportunistic infection in patients with acquired immunodeficiency syndrome (AIDS). It is known that PCP can be treated by the administration of pentamidine by intravenous (iv) infusion or intramuscular (im) injection, although such treatment often causes severe toxic reactions including hypotension, renal failure and hypoglycemia.

The treatment of PCP in rats with aerosolised pentamidine is described in Antimicrob Agents & Chemother, 1987, 31, 37-41 and in Am Rev Respir Dis, 1987, 135, 731-7.

However, these two papers disclose the use of pentamidine at a daily dose of 5 mg/kg, equivalent to a dose for a 70 kg human of 350 mg/day, delivered as an aerosol cloud with a mass median aerodynamic diameter of 2.38 µm. Surprisingly, we have found that PCP can be treated by administering to patients an aerosol spray of pentamidine having a mass median aerodynamic diameter of 4.7 microns (4.7 µm).

Accordingly, one embodiment of the present invention is for a composition for the prophylaxis or treatment of PCP which comprises an aerosol spray of pentamidine or a pharmaceutically acceptable salt thereof as active ingredient, wherein the aerosol spray has a mass median aerodynamic diameter of 4.7 microns (4.7 µm).

The present invention is also for a composition comprising 1 to 300mg of pentamidine or a pharmaceutically acceptable salt thereof intended for administration in the form of an aerosol for the prophylaxis and/or treatment of PCP. In a preferred embodiment, the composition intended for the prophylaxis of PCP contains 10 to 200mg, suitably 30 to 120mg and preferably 45 to 75mg, of pentamidine or a pharmaceutically acceptable salt thereof.

This composition may be in the form of a unit dose presentation, for example an ampoule or vial, containing 1 to 300mg, and preferably 10 to 200mg, of pentamidine or a pharmaceutically acceptable salt in a form readily soluble in water, which unit dose is dissolved in water or saline for nebulisation, or alternatively in the form of a unit dose presentation of a stable solution containing 1 to 300mg, and preferably 10 to 200mg, of pentamidine or a pharmaceutically acceptable salt thereof, which is suitable for nebulisation, with or without further dilution.

We also provide the use of pentamidine or a pharmaceutically acceptable salt thereof for the manufacture of a composition for the prophylaxis or treatment of pneumocystis carinii pneumonia the composition comprising an aerosol spray of pentamidine or a pharmaceutically acceptable salt thereof as active ingredient, wherein the aerosol spray contains significant amounts of particles having a mass median aerodynamic diameter of 4.7 microns (4.7 µm), to a patient susceptible to or suffering from pneumocystis carinii pneumonia.

The present invention is also for the prophylaxis of PCP by the aerosol administration of pentamidine or a pharmaceutically acceptable salt thereof, characterised by the use of a dose level of 1 to 200mg, and suitably 30 to 120mg, of pentamidine or a pharmaceutically acceptable salt thereof. The dose level used is preferably of the order of 45 to 75mg; a specially preferred dose level is 60mg.

In the case of treatment of PCP where the infection is present, higher initial dose levels may be required, and initial dose levels of up to 300mg of pentamidine or a pharmaceutically acceptable salt thereof may be required.

Pneumocystis carinii is frequently present in the lungs, but PCP only develops where there is a defect in the immune system. Prophylaxis of PCP is achieved by the suppression of pneumocystis carinii in the lungs. To achieve this, the inhaled aerosol spray of pentamidine or a pharmaceutically acceptable salt requires to be of small particle size which reach the parts of the lungs susceptible to infection.

Pentamidine is the compound p,p¹-(pentamethylene dioxy) dibenzamidine, which is usually employed in the form of a pharmaceutically acceptable salt. The most commonly available salt is the isethionate, which is of the formula:- 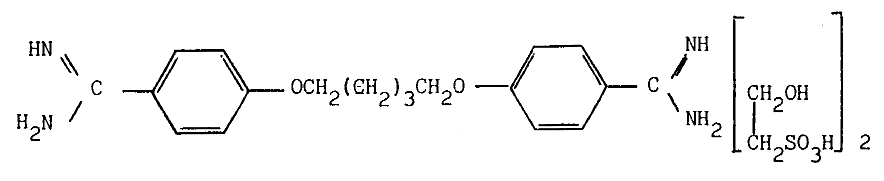
Other salts may also be used such as the hydrochloride or the napthoate. According to a preferred embodiment, pentamidine is used in the form of pentamidine isethionate.

The term aerosol spray as used in this specification means the spray obtained by the nebulisation of an aqueous solution of pentamidine and its salts. Various types of nebulisers are known and used including pneumatic nebulisers and ultrasonic nebulisers. The nebuliser used should be one which delivers significant amounts of particles (or droplets) of solution of a size in the range 0.5 to 8 microns (µm), and preferably 1 to 6 microns (µm). A very suitable ultrasonic nebuliser is the FISONEB nebuliser or Medix Electronic Nebuliser. It is generally preferred to use an ultrasonic nebuliser as the administration period is shorter; delivery times with pneumatic nebulisers are longer, although finer sprays may be obtained.

While the aerosol spray is normally administered by oral inhalation, this may also be administered by intranasal inhalation.

The solution of pentamidine and its salts used for nebulisation is an aqueous solution suitably prepared by dissolving this in sterile pyrogen-free water or saline. However, other aqueous pharmaceutically acceptable carriers may be used.

The concentration of the solution of pentamidine and its salts used for nebulisation may vary from 1 to 100mg/ml, and is suitably 10 to 60mg/ml, and preferably 15 to 20mg/ml.

The amount of pentamidine and its salts contained in the aerosol which is administered to the subject per treatment for prophylaxis of PCP is typically between 10 and 100mg, and suitably 45 to 75mg, and especially about 60mg. While a single treatment may be effective to prevent infection by pneumocystis carinii, it is generally preferable to continue administration on a regular basis to prevent infection. The treatments may be weekly, or every 3 or 4 days, or over other longer or shorter time periods. It is very desirable at the commencement of treatment to build up adequate tissue levels as quickly as possible, and thereafter to maintain the tissue levels of pentamidine. Thus it has been found advantageous to commence the treatment with pentamidine with five administrations each separated by between 24 and 72 hours, and thereafter to have one administration per week, each administration suitably being of a dose of 60mg of pentamidine isethionate.

The compositions of the invention may be prepared in conventional ways.

In the simplest form, the composition comprises an ampoule or vial containing a readily soluble form of the pentamidine or a pharmaceutically acceptable salt thereof, in amount from 1 to 300mg, suitably from 10 to 200mg, and preferably 30 to 120mg, packaged with instructions for use in a nebuliser. The composition may also comprise an ampoule or vial containing sterile water or saline, to facilitate the preparation of the solution for nebulisation. The pentamidine is suitably in the form of a lyophilised powder of pentamidine isethionate.

The composition may also comprise an ampoule or vial containing a stable sterile solution of pentamidine or a pharmaceutically acceptable salt thereof, suitably pentamidine isethionate, in amount comprising 1 to 10ml, and preferably 3ml, such solution containing 1 to 6% by weight of the pentamidine or a salt thereof. The composition preferably comprises an ampoule containing a stable sterile solution of pentamidine isethionate, in amount comprising 3ml, such solution containing 2% by weight pentamidine isethionate.

The following examples are given to illustrate the invention.

### Example 1

A hand-held ultrasonic nebuliser (Siemans Micro-Inhalator 8, model no. TV 7000) was used to administer aerosol pentamidine isethionate to patients with AIDS or ARC (AIDS related complex) as defined by the Centers for Disease Control (ie patients with a reliably diagnosed disease indicative of an underlying cellular immune deficiency).

Each aerosol treatment was given over 20 to 60 minutes, delivering 3.0ml of solution containing from 30 to 60mg pentamidine isethionate, as identified below.

In the first test, twelve patients were treated with bi-weekly doses of 30mg of aerosol pentamidine. The treatment period was three months; patients were examined before and after each treatment and underwent pulmonary function testing and biochemical and hematologic tests before the first treatment and after the first, second, fourth and sixth treatment. No adverse reactions were seen, all patients had stable or improving pulmonary function, and no patient had a proven or possible episode of PCP.

In the second test, a total of 60 patients received fortnightly doses of 30mg of aerosol pentamidine. A total of 650 treatments were given. Treatment periods ranged from 1 to 12 months. No proven adverse reactions have been observed. A single patient developed a severe skin rash after his fifth dose. He had no reactions after his first four doses and was receiving several other medications at the time the rash developed. His rash has since disappeared.

Five proven episodes of PCP have occurred among patients who received bi-weekly doses of 30mg; six proven episodes of PCP have occurred among patients who were treated with this dose, but who missed one or more scheduled doses.

In the third test, patients were randomized to receive doses of 45 or 60mg of aerosol pentamidine. Treatments are administered weekly for the first four weeks and then fortnightly. This new schedule was designed to more rapidly establish protective levels of pentamidine. A total of 73 patients were treated with approximately equal numbers at each dose level. To date two patients receiving 45mg have had proven episodes of PCP. Both were very mild episodes.

In the fourth test, 115 patients received a 60mg dose weekly for 4 weeks and fortnightly thereafter for a total of 590 man months of therapy. Of these patients, 9 had proven episodes of PCP.

### Example 2

A hand-held ultrasonic nebuliser (FISONEB - manufactured by Medix Ltd), which has the characteristic of delivering the majority of particles of an aerodynamic diameter of 0.5 to 8.0 microns (µm) with a mass median aerodynamic diameter of 4.7 microns (µm), was used to administer aerosol pentamidine isethionate to patients with AIDS or ARC as defined by the Centres for Disease Control. Each aerosol treatment was given over 20 to 30 minutes, delivering 3.0ml of solution containing 60mg pentamidine isethionate.

A total of 162 patients were treated for periods ranging from 1 to 8 months (mean 7.1 months); 97% of patients had AIDS; 88% had had PCP; 66% received AZT. A dose of 60mg of pentamidine isethionate was administered weekly for four weeks and then fortnightly.

Pulmonary diffusion capacity improved in 25%, remained stable in 69%, and declined in 6% of patients. A total of 14.7% of patients required pretreatment with a bronchodilator. No other significant adverse reactions were seen. Four mild cases of PCP occurred. The rate of PCP per 100 patient months was 0.35 among patients treated with this regimen; the rate per 100 patient months was 3.4 in an earlier trial using the same dose and a less efficient nebulizer and was 4.6 in patients post first episode of PCP who were treated with AZT while denied prophylaxis for PCP.

## Claims (Claims for the following Contracting State(s): AT, BE, CH, DE, FR, GB, IT, LI, LU, NL, SE)

1. A composition for the prophylaxis or treatment of Pneumocystis carinii pneumonia which comprises an aerosol spray of pentamidine or a pharmaceutically acceptable salt thereof as active ingredient characterised in that the aerosol spray particles have a mass median aerodynamic diameter of 4.7 microns (4.7 µm).

2. A composition according to claim 1 wherein the active ingredient is pentamidine isethionate.

3. A composition according to any one of the preceding claims, wherein the aerosol spray is prepared from a solution of pentamidine or a salt thereof in water or saline solution.

4. A composition according to any one of the preceding claims, wherein the aerosol spray is produced by an ultrasonic nebuliser.

5. Use of pentamidine or a pharmaceutically acceptable salt thereof for the manufacture of a composition for prophylaxis or treatment of Pneumocystis carinii pneumonia, the composition comprising an aerosol spray of pentamidine or a pharmaceutically acceptable salt thereof as active ingredient, wherein the aerosol spray particles have a mass median aerodynamic diameter of 4.7 microns, to a patient susceptible to or suffering from Pneumocystis carinii pneumonia.

6. Use according to claim 5, wherein the aerosol spray is prepared from a solution of the active ingredient in sterile water or saline solution.

7. Use according to any of Claims 5 or 6, wherein the active ingredient is pentamidine isethionate.

## Claims (Claims for the following Contracting State(s): ES, GR)

1. A method for preparing a composition for the prophylaxis or treatment of Pneumocystis carinii pneumonia which comprises producing an aerosol spray from a solution of pentamidine or a pharmaceutically acceptable salt thereof as active ingredient characterised in that the aerosol spray particles have a mass median aerodynamic diameter of 4.7 microns (4.7 µm).

2. A method according to claim 1, wherein the active ingredient is pentamidine isethionate.

3. A method according to any one of the preceding claims, wherein the aerosol spray is prepared from a solution of pentamidine or a salt thereof in water or saline solution.

4. A method according to any one of the preceding claims, wherein the aerosol spray is produced by an ultrasonic nebuliser.

## Patentansprüche (Patentansprüche für folgende(n) Vertragsstaat(en): AT, BE, CH, DE, FR, GB, IT, LI, LU, NL, SE)

1. Zusammensetzung zur Prophylaxe oder Behandlung von Pneumocystis carinii-Pneumonie, welche einen Aerosol-Spray aus Pentamidin oder einem pharmazeutisch annehmbaren Salz hievon als aktiven Bestandteil umfaßt, dadurch gekennzeichnet, daß die Aerosol-Spray-Teilchen einen mittleren aerodynamischen Massedurchmesser von 4,7 Mikron (4,7 µm) aufweisen.

2. Zusammensetzung nach Anspruch 1, worin der aktive Bestandteil Pentamidinisethionat ist.

3. Zusammensetzung nach einem der vorhergehenden Ansprüche, worin der Aerosol-Spray aus einer Lösung von Pentamidin oder eines Salzes hievon in Wasser oder Kochsalzlösung hergestellt ist.

4. Zusammensetzung nach einem der vorhergehenden Ansprüche, worin der Aerosol-Spray durch einen Ultraschall-Vernebler hergestellt ist.

5. Verwendung von Pentamidin oder eines pharmazeutisch annehmbares Salzes hievon zur Herstellung einer Zusammensetzung für die Prophylaxe oder Behandlung von Pneumocystis carinii-Pneumonie, wobei die Zusammensetzung einen Aerosol-Spray aus Pentamidin oder einem pharmazeutisch annehmbaren Salz hievon als aktiven Bestandteil umfaßt, worin die Aerosol-Spray-Teilchen einen mittleren aerodynamischen Massedurchmesser von 4,7 Mikron aufweisen, bei einem Patienten, der für Pneumocystis carinii-Pneumonie anfällig ist oder daran leidet.

6. Verwendung nach Anspruch 5, wobei der Aerosol-Spray aus einer Lösung des aktiven Bestandteils in sterilem Wasser oder Kochsalzlösung hergestellt ist.

7. Verwendung nach Anspruch 5 oder 6, wobei der aktive Bestandteil Pentamidinisethionat ist.

## Patentansprüche (Patentansprüche für folgende(n) Vertragsstaat(en): ES, GR)

1. Verfahren zur Herstellung einer Zusammensetzung für die Prophylaxe oder Behandlung von Pneumocystis carinii-Pneumonie, welches das Erzeugen eines Aerosol-Sprays aus einer Lösung von Pentamidin oder eines pharmazeutisch annehmbaren Salzes hievon als aktiven Bestandteil umfaßt, dadurch gekennzeichnet, daß die Aerosol-Spray-Teilchen einen mittleren aerodynamischen Massedurchmesser von 4,7 Mikron (4,7 µm) aufweisen.

2. Verfahren nach Anspruch 1, bei welchem der aktive Bestandteil Pentamidinisethionat ist.

3. Verfahren nach einem der vorhergehenden Ansprüche, bei welchem der Aerosol-Spray aus einer Lösung von Pentamidin oder eines Salzes hievon in Wasser oder Kochsalzlösung hergestellt wird.

4. Verfahren nach einem der vorhergehenden Ansprüche, bei welchem der Aerosol-Spray durch einen Ultraschall-Vernebler hergestellt wird.

## Revendications (Revendications pour l'(les) Etat(s) contractant(s) suivant(s): AT, BE, CH, DE, FR, GB, IT, LI, LU, NL, SE)

1. Composition pour la prophylaxie ou le traitement d'une pneumonie par Pneumocystis carinii, qui comprend un aérosol de pulvérisation de pentamidine ou d'un sel pharmaceutiquement acceptable de celle-ci comme ingrédient actif, caractérisée en ce que les particules de l'aérosol de pulvérisation ont un diamètre aérodynamique moyen en masse de 4,7 µm.

2. Composition suivant la revendication 1, dans laquelle l'ingrédient actif est l'iséthionate de pentamidine.

3. Composition suivant l'une quelconque des revendications précédentes, dans laquelle l'aérosol de pulvérisation est préparé à partir d'une solution de pentamidine ou d'un sel de celle-ci dans de l'eau ou une solution saline.

4. Composition suivant l'une quelconque des revendications précédentes, dans laquelle l'aérosol de pulvérisation est produit par un nébuliseur à ultrasons.

5. Utilisation de pentamidine ou d'un sel pharmaceutiquement acceptable de celle-ci pour la préparation d'une composition pour la prophylaxie ou le traitement d'une pneumonie par Pneumocystis carinii, la composition comprenant un aérosol de pulvérisation de pentamidine ou d'un sel pharmaceutiquement acceptable de celle-ci comme ingrédient actif, dans laquelle les particules de l'aérosol de pulvérisation ont un diamètre aérodynamique moyen en masse de 4,7 µm, à un patient susceptible de souffrir d'une pneumonie par Pneumocystis carinii.

6. Utilisation suivant la revendication 5, dans laquelle l'aérosol de pulvérisation est préparé à partir d'une solution de l'ingrédient actif dans de l'eau ou une solution saline stérile.

7. Utilisation suivant la revendication 5 ou 6, dans laquelle l'ingrédient actif est l'iséthionate de pentamidine.

## Revendications (Revendications pour l'(les) Etat(s) contractant(s) suivant(s): ES, GR)

1. Procédé de préparation d'une composition pour la prophylaxie ou le traitement d'une pneumonie par Pneumocystis carinii, qui comprend la production d'un aérosol de pulvérisation à partir d'une solution de pentamidine ou d'un sel pharmaceutiquement acceptable de celle-ci comme ingrédient actif, caractérisé en ce que les particules de l'aérosol de pulvérisation ont un diamètre aérodynamique moyen en masse de 4,7 µm.

2. Procédé suivant la revendication 1, dans lequel l'ingrédient actif est l'iséthionate de pentamidine.

3. Procédé suivant l'une quelconque des revendications précédentes, dans lequel l'aérosol de pulvérisation est préparé à partir d'une solution de pentamidine ou d'un sel de celle-ci dans de l'eau ou une solution saline.

4. Procédé suivant l'une quelconque des revendications précédentes, dans lequel l'aérosol de pulvérisation est produit par un nébuliseur à ultrasons.
